Europäisches Patentamt

European Patent Office

Office européen des brevets

(11) Veröffentlichungsnummer: **0 137 297**
**A2**

## (12) EUROPÄISCHE PATENTANMELDUNG

(21) Anmeldenummer: **84110486.2**

(22) Anmeldetag: **04.09.84**

(51) Int. Cl.⁴: **A 61 K 9/22**
**A 61 L 15/04**

(30) Priorität: **24.09.83 DE 3334595**

(43) Veröffentlichungstag der Anmeldung:
**17.04.85 Patentblatt 85/16**

(84) Benannte Vertragsstaaten:
**AT BE CH DE FR GB IT LI NL SE**

(71) Anmelder: **Merck Patent Gesellschaft mit beschränkter Haftung**
**Frankfurter Strasse 250**
**D-6100 Darmstadt(DE)**

(71) Anmelder: **INTERMEDICAT GMBH**
**Gerliswilstrasse 74**
**CH-6020 Emmenbrücke(CH)**

(72) Erfinder: **Fleckenstein, Peter, Dr.**
**Am Bürgerhaus 4**
**D-3501 Fuldabrück 1(DE)**

(72) Erfinder: **Wahlig, Helmut, Dr.**
**Roemheldweg 16**
**D-6100 Darmstadt(DE)**

(72) Erfinder: **Dingeldein, Elvira, Dr.**
**Am Spitzenpfad 9**
**D-6072 Dreieich(DE)**

(54) **Wirkstoffdepot.**

(57) Die Erfindung betrifft ein resorbierbares Wirkstoffdepot auf Basis von Kollagen, wobei ein Kollagen, in dem weitgehend die Porenstruktur des natürlichen Materials erhalten ist, mit einem Aminoglykosid-Antibiotikum und einem bioresorbierbaren Polymeren mit polyanionischem Charakter beladen ist.

Croydon Printing Company Ltd.

EP 0 137 297 A2

B. Braun Melsungen AG

3508 M e l s u n g e n

Merck Patent Gesellschaft
mit beschränkter Haftung

6100 D a r m s t a d t

Wirkstoffdepot

Die Erfindung betrifft ein resorbierbares Wirkstoffdepot
auf Basis von Kollagen.

Es sind bereits zahlreiche Materialien als Träger für
medizinische Wirkstoffe vorgeschlagen worden, die in den
Körper eingebracht werden können und dort während oder
nach der Freisetzung des Wirkstoffs resorbiert werden,
so daß eine nachträgliche Entfernung des Trägers entfallen kann. In der Regel sind dies synthetische organische Polymere, es sind aber auch Implantate auf Basis
von Kollagen vorgeschlagen worden.

So sind in der DOS 28 43 963 im Körper resorbierbare geformte Massen beschrieben, wobei pulverförmiges denaturiertes Kollagen zusammen mit einem Wirkstoff und einem

PAT LOG 5/2 2706

- 2 -

Bindemittel zu Formkörpern verpreßt ist. In der EP-OS 69 260 wird eine wirkstoffhaltige blattförmige Kollagen-Einlage beschrieben, die gegebenenfalls zu einer Stabform aufgewickelt wird. Zur Herstellung dieses Kollagens wird eine Kollagen-Lösung verwendet, die gefriergetrocknet wird.

Alle diese Zubereitungen sind jedoch noch nicht optimal in bezug auf die Freisetzung des Wirkstoffs, die Verträglichkeit und das Resorptionsverhalten des Trägers. Es bestand deshalb die Aufgabe, ein Wirkstoffdepot zu finden, mit dem insbesondere Aminoglykosid-Antibiotika so in den Körper eingebracht werden können, daß der Wirkstoff über einen ausreichend langen Zeitraum in ausreichend hoher Konzentration freigesetzt wird, wobei das Depot gut verträglich ist und gut resorbiert wird.

Es wurde nun gefunden, daß in das Hohlraumsystem eines Kollagens, in dem weitgehend die Porenstruktur des natürlichen Materials erhalten ist, und das über ein relativ hohes Porenvolumen verfügt, Wirkstoffe eingelagert werden können, die beim Einbringen des Kollagens in den Körper freigesetzt werden. Es wurde auch gefunden, daß eine verzögerte Freisetzung insbesondere vom Aminoglykosid-Antibiotika dadurch erreicht werden kann, daß man zusätzlich ein saures Polysaccharid einbringt.

Gegenstand der Erfindung ist daher ein resorbierbares Wirkstoffdepot auf Basis von Kollagen, das dadurch gekennzeichnet ist, daß ein Kollagen, in dem weitgehend die Porenstruktur des natürlichen Materials erhalten ist, mit einem Aminoglykosid-Antibiotikum und einem sauren Polysaccharid beladen ist.

- 3 -

Gegenstand der Erfindung ist auch ein Verfahren zur Herstellung eines resorbierbaren Antibiotikum-Depots, das dadurch gekennzeichnet ist, daß ein Kollagen, in dem weitgehend die Porenstruktur des natürlichen Materials erhalten ist, mit einem Aminoglykosid-Antibiotikum und einem sauren Polysaccharid beladen wird.

Das Ausgangsmaterial zur Herstellung des erfindungsgemäßen Depots kann z. B. nach dem Verfahren des DBP 28 54 490 hergestellt werden.

Das nach diesem Verfahren insbesondere aus Kalbsknochen hergestellte sterile Produkt stellt einen hochporösen Festkörper von gelblich weißer Farbe dar, der praktisch geruchs- und geschmacksfrei ist und beim Anfeuchten in einen schwammartigen Zustand übergeht. In der Porenstruktur, die auch in diesem Zustand im wesentlichen unverändert bleibt, ist weitgehend die Struktur des natürlichen Knochens erhalten, so daß auch das Porenvolumen und die Porengrößenverteilung gewissen Schwankungen unterliegt. Das Material wird jedoch so ausgewählt, daß es ein Porenvolumen von etwa 60 bis etwa 95 %, insbesondere etwa 80 % besitzt. Das Material besitzt in der Regel Dichten zwischen etwa 0,07 und etwa 0,26 $g/cm^3$ und eine Wasseraufnahmekapazität von etwa 500 bis 1500 %. Die Auflösung des Materials in lösliche Bruchstücke erfolgt erst bei extremen pH-Werten infolge hydrolytischer Spaltung oder in Anwesenheit proteolytischer Enzyme, wie z.B. Bakterienkollagenase.

Andere Verfahren zur Herstellung eines porösen Kollagens sind dem Fachmann bekannt und können in analoger Weise zur Gewinnung eines geeigneten Ausgangsmaterials herangezogen werden.

PAT LOG 5/2 2706

Zur Beladung mit dem Wirkstoff wird das Material zweckmäßig mit einer wässerigen Lösung des Wirkstoffs getränkt und danach getrocknet. Durch die Konzentration des Wirkstoffs in der Lösung kann der Gehalt des Wirkstoffs in der Matrix gesteuert werden. Gegebenenfalls kann das Tränken mit Wirkstoff und anschließendes Trocknen auch mehrmals durchgeführt werden, um höhere Gehalte an Wirkstoff zu erzielen. Es werden so in der Regel etwa 1 - 10 Gew.% Wirkstoff in die Kollagen-Matrix eingelagert.

Als Wirkstoffe können im Prinzip alle Stoffe verwendet werden, mit denen Vorgänge im menschlichen oder tierischen Körper beeinflußt werden können. Im Rahmen der vorliegenden Erfindung werden bevorzugt Aminoglykosid-Antibiotika eingelagert, insbesondere bevorzugt wird Gentamycin verwendet.

Aminoglykosid-Antibiotika werden in der Regel in Form ihrer leicht wasserlöslichen Salze, wie z.B. dem Sulfat gehandhabt. Eine Kollagen-Matrix, die z.B. mit Gentamycinsulfat beladen ist, gibt den Wirkstoff beim Einbringen in den Körper relativ schnell ab. Es werden dadurch zwar sehr hohe Konzentrationen an Antibiotikum erzeugt, das Depot ist jedoch nach kurzer Zeit erschöpft. Für die Behandlung z.B. einer infizierten Knochenhöhle ist jedoch die Aufrechterhaltung einer wirksamen Konzentration über einen längeren Zeitraum erwünscht.

Es sind schwer lösliche Derivate von Aminoglykosid-Antibiotika bekannt, wie z.B. das Hesperidin-Phosphat, das Laurylsulfat, das Pamoat oder die aus der DOS 32 06 725 bekannten Flavanoid-Phosphate. Eine Beladung der Kollagen-Matrix mit diesen schwer löslichen Derivaten führt zu einer deutlichen Retardierung der Freisetzung, so daß solche Depots durchaus vorteilhaft eingesetzt werden können.

Es wurde nun jedoch gefunden, daß eine sehr wirksame Retardierung mit einem günstigen Freisetzungsprofil erreicht werden kann, wenn die mit einem leicht löslichen Salz beladene Matrix zusätzlich mit einem sauren Polysaccharid beladen wird. Saure Polysaccharide im Sinne dieser Anmeldung sind polyfunktionelle, physiologisch verträgliche und im Körper resorbierbare Polysaccharidsäuren mit Molekulargewichten bis zu mehreren hunderttausend, die eine Vielzahl von sauren, d.h. ionisierbaren Carboxyl- und Sulfatgruppen enthalten, wie z.B. Pektinsäure, Alginsäure und Carraghenin. Diese Säuren stellen Polyanionen dar, die in der Lage sind, die basischen Aminoglykoside zu präzipitieren. Es werden dabei auch schwer lösliche Derivate der Aminoglykosid-Antibiotika erhalten, wobei die Retardwirkung nicht ausschließlich an die Schwerlöslichkeit geknüpft ist, sondern bereits infolge der multiplen ionischen Wechselwirkungen die Diffusionsrate des Wirkstoffs herabgesetzt wird. Polysaccharidsäuren bewirken schon allein aufgrund ihres ausgeprägten Quellvermögens und ihrer filmbildenden Eigenschaften eine gewisse Retardierung, so daß sich diese Substanzklasse als besonders vorteilhaft bei der Herstellung von Retardformen für Aminoglykoside erweist, da sie in optimaler Weise verschiedene Wirkprinzipien in sich vereint.

- 6 -

Als polyanionische Verbindungen, die diese Merkmale in sich vereinen, kommen saure Polysaccharide aus der Gruppe pflanzlicher Pektinstoffe, die mit unterschiedlichem Veresterungsgrad beispielsweise im Obst vorkommen, saure Kohlehydrate aus Algen, Flechten und anderen niederen Pflanzen sowie andere, freie Carboxyl- und/oder Monoester der Schwefelsäure enthaltende Polysaccharide aus dem Tier-und Pflanzenreich, wie Chondroitinsulfat, Dermatansulfat, Heparansulfat, Hyaluronsäure u.a. in Frage.

Es kommen aber auch Polysäuren auf Basis oxydierter Zellulose, oxydierter Stärke und andere auf synthetischem Wege mit sauren Funktionen ausgestattete makromolekulare Kohlehydrate in Frage. Auch natürliche Polyphosphate, wie sie beispielsweise in den Nukleinsäuren vorliegen, sind prinzipiell geeignet.

Alle diese Stoffe sollen im Sinne der vorliegenden Erfindung unter dem Begriff "saure Polysaccharide" zu verstehen sein. Es liegt auf der Hand, daß der Eignungsgrad unterschiedlicher unter diese Definitionen fallender Stoffe auch unterschiedlich ist. Der Fachmann kann jedoch leicht unter den zugänglichen Materialien solche auswählen, die bezüglich Biokompatibilität, Resorbierbarkeit, gelierender und filmbildender Wirkung und der Retardwirkung auf medizinische Wirkstoffe geeignet sind. Als besonders gut geeignet haben sich Pektinsäure, Alginsäure und Carraghenin erwiesen.

Die erfindungsgemäßen Polysaccharidsäuren sind in Form ihrer Alkalisalze wasserlöslich und bilden je nach ihrer Konzentration mehr oder minder viskose Lösungen. Bringt man die wässerige Lösung eines solchen makromolekularen Stoffes mit der wässerigen Lösung eines Wirkstoffs mit basischen Gruppen zusammen, so kommt es je nach dem pH-Wert der Lösung zur Präzipitation. Während bei pH 9 in der Regel noch keine Präzipitation zu beobachten ist, beginnt diese meist bei etwa pH 8 und verstärkt sich bei niedrigeren pH-Werten.

Bei Werten unterhalb von 5 nimmt die Löslichkeit der Polysaccharide selbst deutlich ab, so daß es dann auch in Abwesenheit eines Wirkstoffs zu gallertartigen Ausscheidungen kommt. Für die Einlagerung des Wirkstoffs in die Kollagenmatrix wird daher der pH-Bereich von 5 - 7 bevorzugt.

Zum Einbringen der Addukte aus Aminoglykosid und Polysäure in die Kollagen-Matrix kann im Prinzip eine vorherige Präzipitation durchgeführt werden, wobei Lösungen von z.B. Gentamycinsulfat und z.B. Natriumalginat bzw. Natriumpektat zusammengegeben und angesäuert werden. Das Präzipitat kann dann mittels Ultraschall in der Matrix verteilt werden. Vorzugsweise wird die Präzipitation jedoch in der Matrix selbst durchgeführt. Dabei wird die Matrix zunächst mit der Lösung eines leicht löslichen Aminoglykosidsalzes und eines wasserlöslichen Alkalisalzes des sauren Polysaccharids beladen. Durch eine pH-Verschiebung auf unterhalb 8, vorzugsweise in den Bereich 5 - 7, wird die Präzipitation im Porensystem der Kollagen-Matrix vervollständigt. Die pH-Verschiebung

- 8 -

kann dabei sowohl in der wässerigen Suspension erfolgen als auch nach Beladen der Matrix mit den beiden Komponenten durch anschließendes Begasen mit den Dämpfen einer genügend flüchtigen, physiologisch unbedenkliche Anionen bildenden Säure, wie z.B. Ameisensäure, Essigsäure- und andere $C_1$-$C_6$-Carbonsäuren. Schließlich kann man die Präzipitation auch durch Immersion in ein organisches, mit Wasser mischbares Lösungsmittel, wie z.B. Aceton oder einen niederen Alkohol wie Methanol, Äthanol oder Propanol, vornehmen, wobei man dem Lösungsmittel die zur Einstellung des gewünschten pH-Wertes erforderliche Menge einer physiologisch akzeptablen Säure zusetzt. Als solche werden Säuren verstanden, die biokompatible Anionen bilden, wie z.B. HCl, $H_2SO_4$, $H_3PO_4$, Ameisensäure, Essigsäure, Milchsäure, Äpfelsäure und andere.

Die Menge des sauren Polysaccharids, die zur Präzipitation des Antibiotikums verwendet wird, richtet sich zum einen nach der Menge des Antibiotikums, nach der Anzahl der basischen Gruppen im Antibiotikum und nach der Anzahl der sauren Gruppen im Polysaccharid. Um eine vollständige Umsetzung des Antibiotikums mit dem Polysaccharid zu erreichen, muß die Menge des Polysaccharids so bemessen sein, daß die Anzahl der sauren Gruppen auf jeden Fall höher ist als die Anzahl der basischen Gruppen des Antibiotikums. Nur beispielhaft genannt werden typische sich daraus ergebende Gewichtsverhältnisse von z.B. Gentamycinbase zu Pektinsäure von etwa 1 zu 1,5 bis etwa 1 zu 15 bzw. von Gentamycinbase zu Alginsäure von etwa 1 zu 1,5 bis etwa 1 zu 7,5.

Diese Mengen können gegebenenfalls jedoch auch unter- oder überschritten werden. Falls die Menge des Polysaccharids nicht ausreicht, um die gesamte Menge an Antibiotika zu binden, wird beim Einbringen des Depots in den Körper zunächst eine relativ hohe Freisetzung erfolgen. Die kann durchaus erwünscht sein, um eine hohe Anfangskonzentration des Antibiotikums zu erzielen, die dann abfällt zu einer wirksamen Dauerdosis.

Andererseits kann durch einen Überschuß an Polysäure eine weitere Retardierung erzielt werden, da das im Porensystem des Kollagens aufquellende Polysaccharid offenbar wie eine Art Ionenaustauscher wirkt, dessen saure Gruppen das aus dem Porensystem herausdiffundierende Antibiotikum reversibel binden und damit die Freisetzung verlangsamen. Es liegt also in der Hand des Fachmanns, das Freisetzungsprofil in weiten Grenzen zu variieren. Durch einen einfachen Vorversuch kann in jedem Fall festgestellt werden, welche Menge des speziellen sauren Polysaccharids zur Präzipitation des speziellen Antibiotikums ausreichend ist.

Es wurde bereits erwähnt, daß die vollständige Präzipitation des Wirkstoff/Polysaccharid-Konjugates nicht nur von der Menge des Polysaccharids, sondern insbesondere auch vom pH-Wert abhängig ist. Da bei physiologischem pH-Wert die Löslichkeit des Konjugates noch recht hoch sein kann, kann es von Vorteil sein, der Kollagen/Wirkstoff-Kombination noch eine Puffersubstanz beizugeben, die im schwach sauren Bereich eine hohe Pufferkapazität besitzt. Auf diese Weise kommt auch im physiologischen Milieu der von den Polysacchariden ausgehende retardierende Effekt voll zum Tragen.

Geeignete Puffersysteme sind z.B. Phosphatpuffer nach Sörensen, Na-acetat/Essigsäure, Na-Lactat/Milchsäure, Na-Malat/Äpfelsäure, Na-Salze saurer Aminosäuren, wie Asparaginsäure und Glutaminsäure, Hydrochloride basischer Aminosäuren, wie Arginin und Lysin, Na-Salze schwacher Peptidsäuren und andere.

Zusätzlich zu den Aminoglykosid-Antibiotika können in die Kollagen-Matrix auch weitere Wirkstoffe eingelagert werden, die eine Antibiotikum-Therapie in sinnvoller Weise ergänzen oder unterstützen können. Auch wenn solche Wirkstoffe keine basischen Gruppen aufweisen, die zu einer Wechselwirkung mit dem sauren Polysaccharid fähig sind, wird auch für diese Wirkstoffe allein aufgrund ihrer Demobilisierung durch die temporäre Blockierung des Porensystems durch die eingelagerten Polysaccharide eine verzögerte Freisetzung aus der Kollagen-Matrix beobachtet.

Nach der Beladung der Kollagen-Matrix mit dem bzw. den Wirkstoffen und dem Polysaccharid wird das Depot gegebenenfalls noch kurz gewaschen, um das bei der doppelten Umsetzung gebildete anorganische Salz, z.B. Natriumsulfat, zu entfernen. Danach wird das Depot isoliert, getrocknet und gegebenenfalls sterilisiert und verpackt.

Das erfindungsgemäße Depot kann zu allen lokalen Antibiotika-Behandlungen in den Körper eingebracht werden und kann aufgrund seiner guten Verträglichkeit und der problemlosen Resorption dort verbleiben. Bevorzugt wird es zur lokalen Bekämpfung von Knocheninfektionen verwendet. Dabei kommt neben der antibakteriellen Wirkung des Antibiotikums der positive Einfluß des natürlichen Kollagens auf den Heilungsprozeß von Knochendefekten zum Tragen.

Das erfindungsgemäße Depot kann als Formstücke unterschiedlicher Geometrie gewonnen werden. Die Kantenlänge kann zwischen etwa 1 und 50 mm variieren, wobei rechteckige Formkörper von ca. 10 mm Kantenlänge bevorzugt werden. Es können aber auch zylindrische, keilförmige, kalottenartige und andere geometrische Formen entsprechend der beabsichtigten Verwendung hergestellt werden. Schließlich kann auch ein unregelmäßig geformtes Granulat mit Teilchengrößen unterhalb von 1 mm gewonnen werden.

Sowohl die Kollagen-Matrix als auch das Polysaccharid werden im Körper bis zur vollständigen Resorption abgebaut, wobei lösliche Fragmente entstehen, die in den normalen körpereigenen Metabolismus eingeschleust werden und keinerlei Nebenwirkungen verursachen.

Durch die vorliegende Erfindung steht daher ein wertvolles neues Wirkstoffdepot zur Verfügung.

Beispiel 1

200 ml einer wässerigen Lösung von 1,70 g Gentamycinsulfat, die mit NaOH auf pH 9 eingestellt ist, wird mit 200 ml einer wässerigen Lösung von 1,70 g Pektinsäure-Natriumsalz (Veresterungsgrad ca. 8 %), die ebenfalls mit NaOH auf pH 9 eingestellt ist, vermischt. In diese Mischung werden 10,0 g eines nach dem Verfahren des DBP 28 54 490 gewonnenen Kollagens in Form von quaderförmigen Teilchen mit den ungefähren Abmessungen 2 x 2 x 1 cm eingetragen. Durch mehrmaliges Evakuieren und anschließendes Belüften wird die Luft aus dem porösen Material entfernt. Unter vorsichtigem Rühren läßt man dann verdünnte, etwa 1 n Schwefelsäure zulaufen, bis die Mischung einen pH-Wert von 6,5 besitzt. Das Gesamtvolumen beträgt danach etwa 500 ml. Die Kollagenstückchen werden dann entnommen und nach kurzem Abtropfen bei -20 °C eingefroren und danach lyophilisiert. Das so gewonnene Wirkstoffdepot, das etwa 2 Gew.% Gentamycinbase enthält, kann durch Bestrahlung und z.B. durch Einsiegelung in Doppel-peel-Täschchen keimfrei verpackt werden.

Beispiel 2

Es wird analog Beispiel 1 verfahren, wobei jedoch anstelle von 1,70 g Gentamycinsulfat bzw. Pektinsäure-Natriumsalz jeweils 4,5 g eingesetzt wird. Man erhält ein Wirkstoffdepot mit etwa 5 Gew.% Gentamycin-Base.

Intermedicat GmbH
CH-6020 Emmenbrücke

**Merck Patent Gesellschaft
mit beschränkter Haftung**

**6100 D a r m s t a d t**

Patentansprüche

1. Resorbierbares Wirkstoffdepot auf Basis von Kollagen, dadurch gekennzeichnet, daß ein Kollagen,
   in dem weitgehend die Porenstruktur des natürlichen
   Materials erhalten ist, mit einem Aminoglykosid-
   Antibiotikum und einem bioresorbierbaren Polymeren
   mit polyanionischem Charakter beladen ist.

2. Wirkstoffdepot nach Anspruch 1, dadurch gekennzeichnet, daß das Kollagen ein Porenvolumen von
   etwa 60 bis etwa 95 % besitzt.

3. Wirkstoffdepot nach Anspruch 1 oder 2, dadurch gekennzeichnet, daß als bioresorbierbares Polymer
   ein saures Polysaccharid enthalten ist.

4. Wirkstoffdepot nach Anspruch 3, dadurch gekennzeichnet, daß als saures Polysaccharid Pektinsäure oder Alginsäure enthalten ist.

5. Wirkstoffdepot nach einem der Ansprüche 1 bis 4, dadurch gekennzeichnet, daß als Antibiotikum Gentamycin enthalten ist.

6. Wirkstoffdepot nach einem der Ansprüche 1 bis 5, dadurch gekennzeichnet, daß das Antibiotikum in einer Menge von 1 - 10 Gew.% enthalten ist.

7. Wirkstoffdepot nach einem der Ansprüche 1 bis 6, dadurch gekennzeichnet, daß das saure Polysaccharid gegenüber dem Aminoglykosid-Antibiotikum im Überschuß vorliegt.

8. Verfahren zur Herstellung eines resorbierbaren Antibiotikum-Depots, dadurch gekennzeichnet, daß ein Kollagen, in dem weitgehend die Porenstruktur des natürlichen Materials erhalten ist, mit einem Aminoglykosid-Antibiotikum und einem bioresorbierbaren Polymeren mit polyanionischem Charakter beladen wird.

9. Verfahren nach Anspruch 8, dadurch gekennzeichnet, daß die Kollagen-Matrix zunächst mit der Lösung eines Salzes eines Aminoglykosid-Antibiotikums und eines Salzes eines sauren Polysaccharids getränkt wird und danach eine pH-Verschiebung in einen Bereich von pH 4 bis 8 vorgenommen wird.

10. Verfahren nach Anspruch 8 oder 9, dadurch gekennzeichnet, daß als Salz eines Aminoglykosid-Antibiotikums Gentamycinsulfat und als Salz eines sauren Polysaccharids Natriumalginat oder Natriumpektat verwendet wird.

11. Verwendung eines Kollagens, in dem weitgehend die Porenstruktur des natürlichen Materials erhalten ist, zur Herstellung eines resorbierbaren Wirkstoffdepots.

PAT LOG 5/1 2706